# EUROPÄISCHE PATENTSCHRIFT

(11) **EP 0 441 747 B1**
(45) Veröffentlichungstag und Bekanntmachung des Hinweises auf die Patenterteilung: **03.07.1996**
(21) Anmeldenummer: 91810066.0
(22) Anmeldetag: 29.01.1991
(51) Int. Cl.: C07D 239/42, A01N 43/54

(54) **Mikrobizide**
Microbicide agent
Agent microbicide

(30) Priorität: 07.02.1990 CH 388/90
(43) Veröffentlichungstag der Anmeldung: 14.08.1991
(73) Patentinhaber: CIBA-GEIGY AG, CH-4002 Basel (CH)
(72) Erfinder: Zondler, Helmut, Dr., CH-4103 Bottmingen (CH)

(56) Entgegenhaltungen:
- EP-A- 0 270 111
- EP-A- 0 337 943

## Beschreibung

Die vorliegende Erfindung betrifft neue Pyrimidinyl-phenylhydroxylamin-Derivate der nachstehenden Formel I. Sie betrifft ferner die Herstellung dieser Substanzen sowie agrochemische Mittel, die als Wirkstoff mindestens eine dieser Verbindungen enthalten. Die Erfindung betrifft ebenso die Herstellung der genannten Mittel sowie die Verwendung der Wirkstoffe oder der Mittel zur Bekämpfung oder Verhütung eines Befalls von Pflanzen durch phytopathogene Mikroorganismen, vorzugsweise Fungi.

Die erfindungsgemässen Verbindungen entsprechen der allgemeinen Formel I 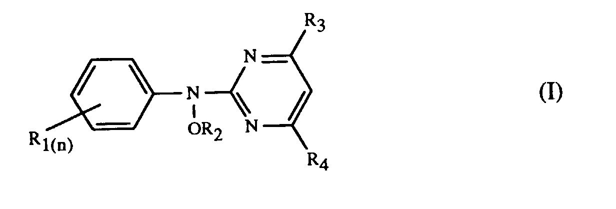 in welcher bedeuten:
R₁ Wasserstoff, Halogen, Methyl, Methoxy oder Trifluormethyl;
n 1 oder 2;
R₂ Wasserstoff, C₁-C₆-Alkyl, mit Halogen, Cyano oder Methoxy substituiertes C₁-C₃-Alkyl, oder C₂-C₅-Alkenyl, C₂-C₅-Alkinyl, Benzyl, COR₅, CON(R₅)R₆, CSN(R₅)R₆, CO(OR₆), CO(SR₆), CS(SR₅), SO₂R₇, PO(OR₆)₂ oder Si(R₆)₃; R₃ und R₄ unabhängig voneinander Wasserstoff, Cyclopropyl, oder Methylcyclopropyl und einer von R₃ und R₄ kann auch C₁-C₅-Alkyl sein;
R₅ Wasserstoff, C₁-C₆-Alkyl, mit 1 bis 3 Halogen substituiertes C₁-C₃-Alkyl, mit OR₆ oder SR₆ substituiertes C₁-C₃-Alkyl, C₂-C₅-Alkenyl, mit 1 bis 3 Halogen substituiertes C₂-C₅-Alkenyl, C₂-C₅-Alkinyl, C₃-C₆-Cycloalkyl, Phenyl oder 1- bis 3-fach mit Halogen, C₁-C₃-Alkyl, C₁-C₃-Halogenalkyl, C₁-C₃-Alkoxy, Nitro oder Cyano substituiertes Phenyl;
R₆ C₁-C₆-Alkyl;
R₇ C₁-C₃-Alkyl, Phenyl, mit Halogen, C₁-C₂-Alkyl, C₁-C₂-Halogenalkyl, Methoxy, Nitro, 1- oder 2-fach substituiertes Phenyl; einschliesslich der Säureadditionssalze der Verbindungen der Formel I.

Unter dem Begriff Alkyl selbst oder als Bestandteil eines anderen Substituenten sind je nach Anzahl der angegebenen Kohlenstoffatome beispielsweise folgende Gruppen zu verstehen: Methyl, Ethyl, Propyl, Butyl, Pentyl oder Hexyl sowie z.B. ihre Isomeren Isopropyl, Isobutyl, tert.-Butyl, sek.-Butyl, Isopentyl usw.. Alkenyl steht z.B. für Propenyl-(1), Allyl, Butenyl-(1), Butenyl-(2) oder Butenyl-(3). Halogen soll hier und im folgenden Fluor, Chlor, Brom oder Jod, vorzugsweise Fluor, Chlor oder Brom, bedeuten.

Die Verbindungen der Formel I sind bei Raumtemperatur stabile Oele, Harze oder Feststoffe, die sich durch sehr wertvolle physiologische, wie mikrobizide, beispielsweise pflanzenfungizide Eigenschaften auszeichnen. Sie lassen sich daher einerseits auf dem Agrogebiet oder verwandten Bereichen zur Bekämpfung von phytopathogenen Mikroorganismen, insbesondere von Fungi, einsetzen.

Die Erfindung betrifft sowohl die freien Verbindungen der Formel I, als auch deren Additionssalze mit anorganischen und organischen Säuren.

Erfindungsgemässe Salze sind insbesondere Additionssalze mit nach Massgabe des Einsatzzweckes physiologisch unbedenklichen anorganischen oder organischen Säuren, wie beispielsweise Halogenwasserstoffsäuren, z.B. Chlor-, Brom- oder Jodwasserstoffsäure, sowie Schwefelsäure, Phosphorsäure, phosphorige Säure, Salpetersäure, gegebenenfalls halogenierte Fettsäuren, wie Essigsäure, Trichloressigsäure und Oxalsäure, oder Sulfonsäuren, wie Benzolsulfonsäure und Methansulfonsäure oder auch Additionssalze mit geeigneten Salzen, wie beispielsweise Magnesium- oder Calciumchlorid.

Folgende Wirkstoffgruppen sind aufgrund ihrer ausgeprägten pflanzenschützenden Eigenschaften bevorzugt:
a) Verbindungen der Formel I, worin bedeuten:
   R₁ Wasserstoff, Fluor, Chlor oder Brom;
   n 1;
   R₂ Wasserstoff, C₁-C₄-Alkyl, C₂-C₃-Alkenyl, C₂-C₃-Alkinyl, COR₅, CON(R₅)R₆, CSNR₅(R₆) oder COOR₆;
   R₃ und R₄ unabhängig voneinander Wasserstoff, Cyclopropyl oder Methylcyclopropyl und einer von R₃ und R₄ kann auch C₁-C₅-Alkyl sein;
   R₅ Wasserstoff, C₁-C₆-Alkyl, C₃-C₆-Cycloalkyl, C₂-C₅-Alkenyl, C₂-C₅-Alkinyl, Phenyl oder einfach mit Fluor, Chlor, Brom, Methyl, Trifluormethyl, Trichlormethyl, Tribrommethyl, Methoxy, Nitro oder Cyano substituiertes Phenyl;
   R₆ C₁-C₃-Alkyl.
b) Verbindungen der Formel I, worin bedeuten:
   R₁ Wasserstoff, Fluor oder Chlor;
   n 1;
   R₂ Wasserstoff, C₁-C₃-Alkyl, COR₅, CON(R₅)R₆, CSNR₅(R₆) oder COOR₆;
   R₃ und R₄ unabhängig voneinander Cyclopropyl oder Methylcyclopropyl und einer von R₃ und R₄ kann auch C₁-C₃-Alkyl sein;
   R₅ Wasserstoff oder C₁-₄Alkyl;
   R₆ C₁-C₃-Alkyl.

Die folgenden Verbindungen zeichnen sich durch besonders vorteilhafte pflanzenschützende Eigenschaften aus:
N-(4-Methyl-6-cyclopropylpyrimidinyl-2)-phenylhydroxylamin (Verb. Nr. 1.13);
N-(4-Methyl-6-cyclopropylpyrimidinyl-2)-3-fluorphenylhydroxylamin (Verb. Nr. 1.16);
O-Acetyl-N-(4-methyl-6-cyclopropylpyrimidinyl-2)-phenylhydroxylamin (Verb. Nr. 1.26);
O-Pivaloyl-N-(4-methyl-6-cyclopropylpyrimidinyl-2)-phenylhydroxylamin (Verb. Nr. 1.36).

Die Verbindungen der Formel I werden hergestellt, indem man umsetzt:
A) ein Pyrimidinylhalogenid der Formel II 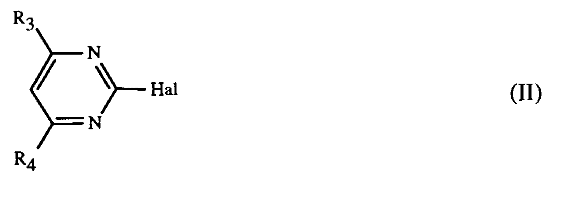 mit einem Phenylhydroxylamin der Formel III 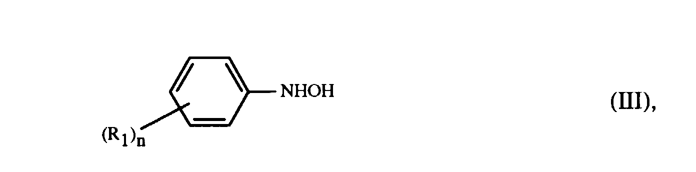 worin R₁, R₃ und R₄ und n die unter Formel I angegebenen Bedeutungen besitzen und Hal ein Halogen, vorzugsweise Chlor oder Brom, darstellt, in Gegenwart einer Säure in inerten organischen Lösungsmitteln bei Temperaturen von -30° bis 100°C, bevorzugt 0° bis 50°C; und
B) die daraus resultierende Verbindung Ia 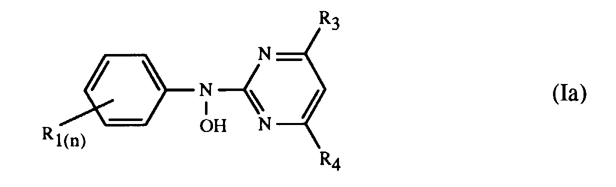
C) mit einer Verbindung der Formel IV

   R₂ - X (IV),

   worin R₁, R₂, R₃, R₄ und n die unter Formel I angegebenen Bedeutungen besitzen und X Halogen, vorzugsweise Chlor oder Brom, oder den Imidazolylrest darstellt; oder
D) mit einem Anhydrid der Formel V

   (R₅CO)₂O (V);

   oder
E) mit einem Isocyanat der Formel VI

   R₅-N=C=Y (VI),

   worin Y Sauerstoff oder Schwefel darstellt, in inerten organischen Lösungsmitteln bei Temperaturen von -20° bis 150°C, vorzugsweise 0° bis 100°C, und bezüglich der Reaktionen (C) und (D) in Gegenwart eines säurebindenen Mittels.

Als Reaktionsmedien in Anpassung an die jeweiligen Reaktionsbedingungen können z.B. folgende Lösungs- und Verdünnungsmittel verwendet werden: aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; ferner Dimethylsulfoxid oder N-Methylpyrrolidon; sowie Gemische solcher Lösungsmittel untereinander.

Als Säuren finden sowohl anorganische als auch organische Säuren Anwendung, wie beispielsweise Halogenwasserstoffsäure, z.B. Fluorwasserstoffsäure, Chlorwasserstoffsäure oder Bromwasserstoffsäure, ferner Schwefelsäure, Phosphorsäure oder Salpetersäure sowie beispielsweise Essigsäure, Ameisensäure, Oxalsäure, Zitronensäure, Trifluoressigsäure, Methansulfonsäure oder Toluolsulfonsäure.

Als säurebindende Mittel dienen Protonenakzeptoren, vornehmlich organische Basen, wie beispielsweise tertiäre Amine, z.B. Triethylamin, Dimethylaminobenzol, Diethylaminobenzol oder Pyridin, sowie organische Basen, wie beispielsweise Alkali- oder Erdalkaliverbindungen, z.B. die Hydroxide, Oxide oder Karbonate von Lithium, Natrium, Kalium, Magnesium, Calcium, Strontium und Barium oder auch Hydride, z.B. Natriumhydrid. Die 2-Halogenpyrimidine der Formel II sind bekannt oder können nach dem Fachmann bekannten Methoden hergestellt werden (Lit. vgl. D.J. Brown, The Pyrimidines in Heterocyclic Compounds, 1962, Interscience Publishers, New York). Im vorstehend beschriebenen Verfahren gemäss Variante (A) werden insbesondere 2-Chlorpyrimidine verwendet.

Die Phenylhydroxylamine der Formel III werden hergestellt durch Reduktion von Nitrobenzolderivaten mit Hydrazinhydrat in Gegenwart von Rhodiumkatalysatoren (Lit., vgl. Oxley, Organic Synthesis 67, 187).

Aus der Literatur sind 2-Phenylamino-4,6-disubstituierte Pyrimidin-Derivate bekannt. Solche Stoffe sind z.B. in den europäischen Patentanmeldungen Nr. 243 136 und Nr. 270 111 als Wirksubstanzen gegen schädliche Mikroorganismen, darunter auch phytopathogene Fungi, beschrieben. Diese Substanzen erfüllen jedoch nicht immer die in der Praxis an sie gestellten Forderungen.

Ueberraschenderweise wurde nun gefunden, dass Verbindungen der Formel I ein für praktische Bedürfnisse sehr günstiges biozides Spektrum zur Bekämpfung von Insekten und phytopathogenen Mikroorganismen, insbesondere Fungi, aufweisen. Sie besitzen sehr vorteilhafte kurative, präventive und insbesondere systemische Eigenschaften und werden zum Schutz von zahlreichen Kulturpflanzen eingesetzt. Mit den Wirkstoffen der Formel I können an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile z.B. vor phytopathogenen Mikroorganismen verschont bleiben.

Verbindungen der Formel I sind z.B. gegen die den folgenden Klassen angehörenden phytopathogenen Pilze wirksam: Fungi imperfecti (insbesondere Botrytis, ferner Pyricularia, Helminthosporium, Fusarium, Septoria, Cercospora und Alternaria); Basidiomyceten (z.B. Rhizoctonia, Hemileia, Puccinia). Darüber hinaus wirken sie gegen die Klasse der Ascomyceten (z.B. Venturia und Erysiphe, Podosphaera, Monilinia, Uncinula) und der Oomyceten (z.B. Phytophthora, Pythium, Plasmopara). Die Verbindungen der Formel I können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Die Erfindung betrifft auch die Mittel, die als Wirkstoffkomponente Verbindungen der Formel I enthalten, insbesondere pflanzenschützende Mittel, sowie deren Verwendung auf dem Agrarsektor oder verwandten Gebieten.

Darüber hinaus schliesst die vorliegende Erfindung auch die Herstellung dieser Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der neuen Verbindungen der Formel I bzw. der neuen Mittel auszeichnet.

Als Zielkulturen für den hierin offenbarten pflanzenschützenden Einsatz gelten im Rahmen dieser Erfindung beispielsweise folgende Pflanzenarten: Getreide (Weizen, Gerste, Roggen, Hafer, Reis, Mais, Sorghum und verwandte Species); Rüben (Zucker- und Futterrüben); Kern-, Stein und Beerenobst (Aepfel, Birnen, Pflaumen, Pfirsiche, Mandeln, Kirschen, Erd-, Him- und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamomum, Kampfer) oder Pflanzen wie Tabak, Nüsse, Kaffee, Zuckerrohr, Tee, Pfeffer, Weinreben, Hopfen, Bananen- und Naturkautschukgewächse sowie Zierpflanzen.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffs der Formel I bzw. eines agrochemischen Mittels, das mindestens einen dieser Wirkstoffe enthält, ist das Aufbringen auf das Blattwerk (Blattapplikation). Applikationsfrequenz und Aufwandmenge richten sich dabei nach dem Befallsdruck des betreffenden Erregers. Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Bei Wasserreiskulturen kann man solche Granulate dem überfluteten Reis-Feld zudosieren. Die Verbindungen der Formel I können aber auch auf Samen- körner aufgebracht werden (Coating), indem man die Körner entweder in einer flüssigen Zubereitung des Wirkstoffs tränkt oder sie mit einer festen Zubereitung beschichtet.

Die Verbindungen der Formel I werden dabei in unveränderter Form oder vorzugsweise zusammen mit den in der Formulierungstechnik üblichen Hilfsmitteln eingesetzt. Dafür werden sie zweckmässigerweise z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 10 g bis 5 kg Aktivsubstanz (AS) je ha, bevorzugt 20 g bis 1 kg AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel kommen in Frage: Aromatische Kohlenwasserstoffe, bevorzugt die Fraktionen C₈ bis C₁₂, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorilonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien, z.B. Calcit oder Sand, in Frage. Darüber hinaus kann eine Vielzahl von vorgranulierten Materialien anorganischer und organischer Natur wie insbesondere Dolomit oder zerkleinerte Pflanzenrückstände verwendet werden.

Besonders vorteilhafte applikationsfördernde Zuschlagstoffe, die zu einer starken Reduktion der Aufwandmenge führen können, sind ferner natürliche (tierische oder pflanzliche) oder synthetische Phospholipide.

Als oberflächenaktive Verbindungen kommen je nach Art des zu formu- lierenden Wirkstoffs der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien genannt die Alkali-, Erdalkali- oder gegebenenfalls substituierten Ammoniumsalze von höheren Fettsäuren (C₁₀-C₂₂), wie z.B. die Na- oder K-Salze der Oel- oder Stearinsäure, oder von natürlichen Fettsäuregemischen, die z.B. aus Kokosnussoder Talgöl gewonnen werden können. Ferner sind auch die Fettsäure-Methyltaurinsalze zu erwähnen.

Häufiger werden jedoch sog. synthetische Tenside verwendet, insbesondere Alkansulfonate, Fettalkoholsulfate, sulfonierte Benzimidazolderivate oder Alkylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na-oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind wasserlösliche 20 bis 250 Aethylenglykoläthergruppen und 10 bis 100 Propylenglykolethergruppen enthaltende Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ammoniumbromid.

Weitere in der Formulierungstechnik gebräuchlichen Tenside sind dem Fachmann bekannt oder können der einschlägigen Fachliteratur entnommen werden.

Die agrochemischen Zubereitungen enthalten in der Regel 0,1 bis 99 %, insbesondere 0,1 bis 95 %, Wirkstoff der Formel I, 99,9 bis 1 %, insbesondere 99,9 bis 5 %, eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele dienen zur näheren Erläuterung der Erfindung, ohne dieselbe einzuschränken.

### 1. Herstellungsbeispiele:

### 1.1 Herstellung von N-(4-Methyl-6-cycloprooylpyrimidinyl-2)-phenylhydroxylamin

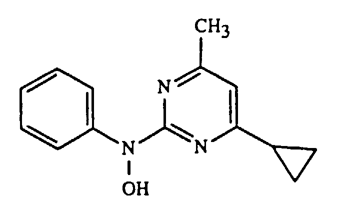

Man löst 4,22 g (0,025 Mol) 2-Chlor-4-methyl-6-cyclopropylpyrimidin mit 3,06 g (0,028 Mol) Phenylhydroxylamin in 25 ml Methanol, fügt 2 ml Essigsäure zu und lässt die Lösung bei Raumtemperatur über Nacht stehen. Im Dünnschichtchromatogramm lässt sich danach kein Ausgangspyrimidin mehr nachweisen. Man giesst das Gemisch in eine Mischung aus Wasser und Essigsäureethylester, rührt und neutralisiert mit Natriumhydrogencarbonat auf pH 7. Abtrennen der organischen Phase, zweimalige Nachextraktion, Trocknung der Extrakte mit Natriumsulfat und Entfernung des Lösungsmittels am Rotationsverdampfer ergibt 6,5 g Rohprodukt, dessen Umkristallisation aus 16 ml Essigsäureethylester 3,77 g (62,5 % d. Theorie) Reinprodukt liefert; Smp. 121,5-123°C. Aus der Mutterlauge erhält man weitere 0,73 g Reinsubstanz, so dass sich die Gesamtausbeute auf 74,6 % d. Th. erhöht.

| Analyse C₁₄H₁₅N₃O (M = 241,29) | | |
|---|---|---|
| | % ber. | % gef. |
| C | 69,69 | 69,83 |
| H | 6,27 | 6,16 |
| N | 17,42 | 16,93 |
| O | 6,63 | 7,13 |

### 1.2 Herstellung von N-(4-Methvl-6-methoxymethylpyrimidinyl-2)-3-fluorphenylhydroxylamin

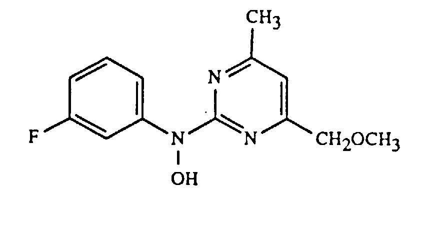

4,32 g (0,025 Mol) 2-Chlor-4-methyl-6-methoxymethylpyrimidin werden in 25 ml Methanol und 2 ml Eisessig bei Raumtemperatur mit 3,81 g (0,03 Mol) 3-Fluorphenylhydroxylamin versetzt. Die Reaktion kommt nur langsam in Gang und wird nach Zugabe von 2 ml konzentrierter wässriger Salzsäure schneller (Dünnschichtchromatographie). Nach dem vollständigen Umsatz des Ausgangspyrimidins wird durch Extraktion mit Wasser und Essigsäureethylester zum Rohprodukt (6,7 g) aufgearbeitet. Dessen chromatographische Aufarbeitung (Kieselgel; Laufmittel 25 Teile Essigsäureethylester und 75 Teile Hexan) ergibt 4,59 g (69,7 % der Theorie) Reinsubstanz als Oel. NMR-Daten siehe Tabelle 2.

| Analyse C₁₃H₁₄FN₃O₂ (M = 263,27) | | |
|---|---|---|
| | % ber. | % gef. |
| C | 59,31 | 59,44 |
| H | 5,36 | 5,52 |
| N | 15,96 | 15,60 |
| O | 7,22 | 7,28 |

### 1.3 Herstellung von O-Propargyl-N-(4-methyl-6-cyclopropylpyrimidinyl-2)-phenylhydroxylamin

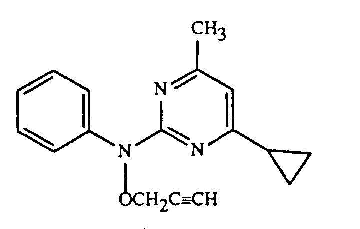

1,32 g (0,0055 Mol) N-(4-Methyl-6-cyclopropylpyrimidinyl-2)-phenylhydroxylamin werden mit 0,72 g (0,006 Mol) Propargylbromid und 0,2 g Cetyltrimethylammoniumbromid in 10 ml Methylenchlorid und 5 ml 30%iger Natronlauge 60 Minuten bei Raumtemperatur gerührt. Die Alkylierung verläuft vollständig (Dünnschichtchromatographie). Die Extraktion mit Wasser und Chloroform führt zu einem Rohprodukt, dessen Reinigung mittels Säulenchromatographie (Kieselgel; Laufmittel ein Gemisch aus 20 Teilen Essigsäureethylester und 80 Teilen Hexan) 1,55 g Reinsubstanz als Oel ergibt. NMR-Daten siehe Tabelle 2.

| Analyse C₁₇H₁₇N₃O (M = 279,34) | | |
|---|---|---|
| | % ber. | % gef. |
| C | 73,10 | 72,91 |
| H | 6,14 | 6,11 |
| N | 15,04 | 14,94 |

### 1.4 Herstellung von O-Methylcarbamyl-N-(4-methyl-6-cyclopropylpyrimidinyl-2)-phenylhydroxylamin

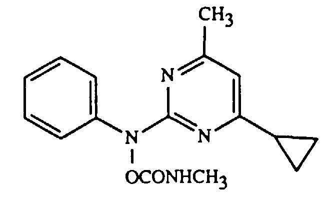

3,14 g (0,013 Mol) N-(4-Methyl-6-cyclopropylpyrimidinyl-2)-phenylhydroxylamin werden mit 0,86 g (0,015 Mol) Methylisocyanat in 40 ml Tetrahydrofuran gelöst und mit einigen Tropfen Triethylamin versetzt. Die Umsetzung erfolgt bei Raumtemperatur innerhalb 1 Stunde. Man entfernt das Lösungsmittel am Rotationsverdampfer und kristallisiert den Rückstand aus einem Gemisch von 4 ml Toluol und 6 ml Cyclohexan um. Ausbeute: 3,60 g (92,7 % d. Theorie); Smp. 96-97°C.

| Analyse C₁₆H₁₈N₄O₂ (M = 298,35) | | |
|---|---|---|
| | % ber. | % gef. |
| C | 64,41 | 64,40 |
| H | 6,08 | 6,15 |
| N | 18,78 | 18,91 |

### 1.5 Herstellung von O-Pronionyl-N-(4-methyl-6-methoxymethylpyrimidinyl-2)-3-fluorphenylhydroxylamin

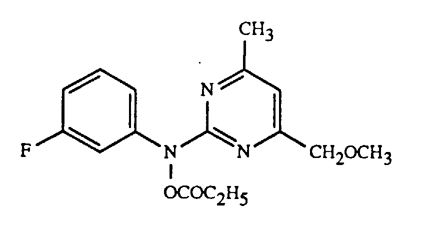

2,49 g (0,0095 Mol) N-(4-Methyl-6-methoxymethylpyrimidinyl-2)-3-fluorphenylhydroxylamin werden mit 1,5 g Triethylamin in 30 ml Tetrahydrofuran gelöst und tropfenweise bei maximal 10°C mit einer Lösung von 1,16 g (0,0125 Mol) Propionsäurechlorid in 10 ml Tetrahydrofuran versetzt. Es scheidet sich Triethylamin-Hydrochlorid ab. Man extrahiert mit Wasser und Chloroform und erhält nach dem Entfernen der Lösungsmittel 3,71 g Rohprodukt, das mittels Säulenchromatographie gereinigt wird. Die Reinausbeute beträgt 1,99 g Oel (65,7 % der Theorie). NMR-Daten siehe Tabelle 2.

| Analyse C₁₆H₁₈FN₃O₃ (M = 319,34) | | |
|---|---|---|
| | % ber. | % gef. |
| C | 60,18 | 59,84 |
| H | 5,68 | 5,60 |
| N | 13,16 | 13,45 |
| F | 5,95 | 6,01 |

### 1.6 Herstellung von O-Diethylcarbamyl-N-(4-methyl-6-cyclopropylpyrimidinyl-2)-3-fluorphenylhydroxylamin

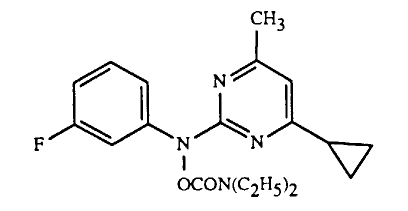

Man löst 2,20 g (0,0085 Mol) N-(4-Methyl-6-cyclopropylpyrimidinyl-2)-3-fluorphenylhydroxylamin mit 1,33 g (0,0098 Mol) Diethylcarbamoylchlorid in 20 ml Tetrahydrofuran und fügt 1,12 g (0,011 Mol) Triethylamin hinzu. Erst nach Zugabe von 0,20 g Dimethylaminopyridin kommt die Umsetzung beim Kochen in Gang und läuft sauber zu Ende. Man extrahiert mit Wasser und Essigsäureethylester, isoliert das Rohprodukt und reinigt es mittels Säulenchromatographie. Ausbeute 3,07 g Oel. NMR-Daten siehe Tabelle 2.

| Analyse C₁₉H₂₃FN₄O₂ | | |
|---|---|---|
| | % ber. | % gef. |
| C | 63,67 | 63,79 |
| H | 6,47 | 6,67 |
| N | 15,63 | 15,36 |
| F | 5,30 | 5,29 |

### 1.7 Herstellung von O-Carbomethoxy-N-(4-methyl-6-cyclopropylpyrimidinyl-2)-3-fluorphenylhydroxylamin

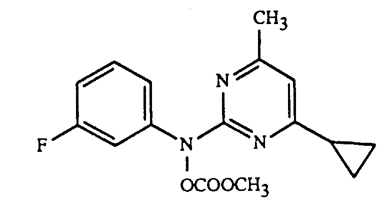

Man löst 3,89 g (0,015 Mol) N-(4-Methyl-6-cyclopropylpyrimidinyl-2)-3-fluorphenylhydroxylamin mit 2,02 g (0,02 Mol) Triethylamin in 20 ml Tetrahydrofuran und lässt unter Kühlung bei Raumtemperatur eine Lösung von 1,56 g (0,0165 Mol) Chlorameisensäuremethylester in 8 ml Tetrahydrofuran zutropfen. Es fällt dabei sofort Triethylaminhydrochlorid aus. Man extrahiert mit Essigsäureethylester und reinigt das Rohprodukt durch Säulenchromatographie auf Kieselgel. Man erhält 4,40 g Produkt, das aus 15 ml n-Hexan und 6 ml Essigsäureethylester umkristallisiert wird. Die Ausbeute beträgt 4,10 g (86 % der Theorie); Smp. 59-60°C.

| Analyse C₁₆H₁₆FN₃O₃ (MG = 317,32) | | |
|---|---|---|
| | % ber. | % gef. |
| C | 60,56 | 60,74 |
| H | 5,08 | 5,18 |
| N | 13,24 | 13,43 |
| F | 5,99 | 6,08 |

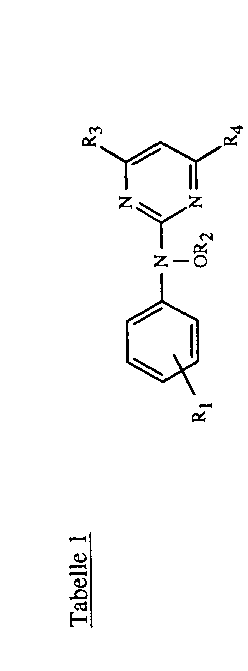

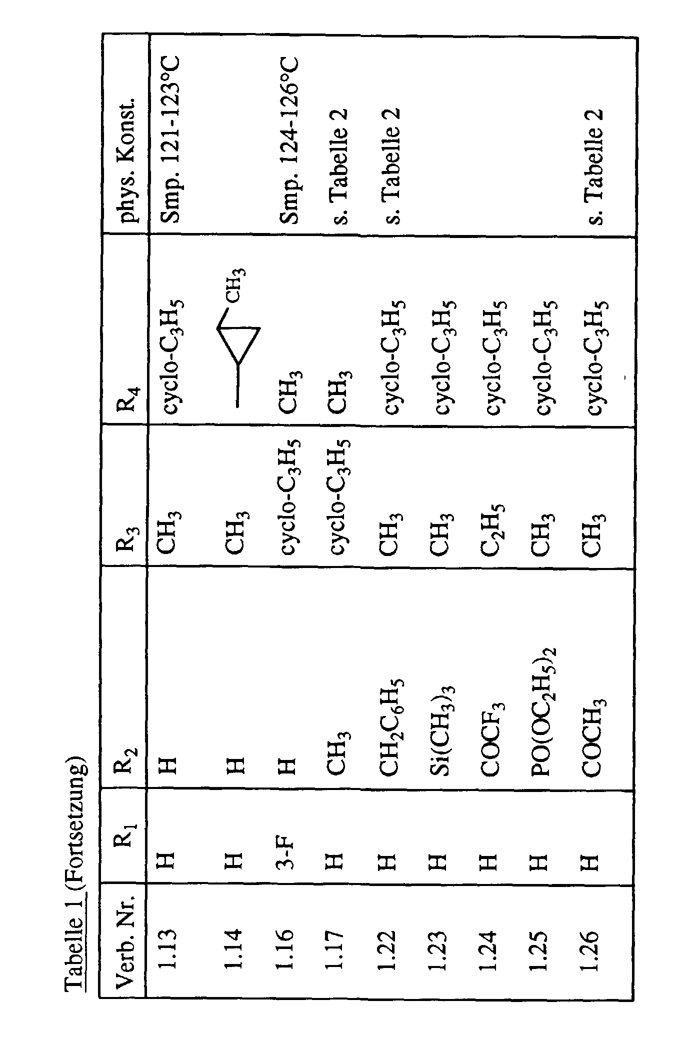

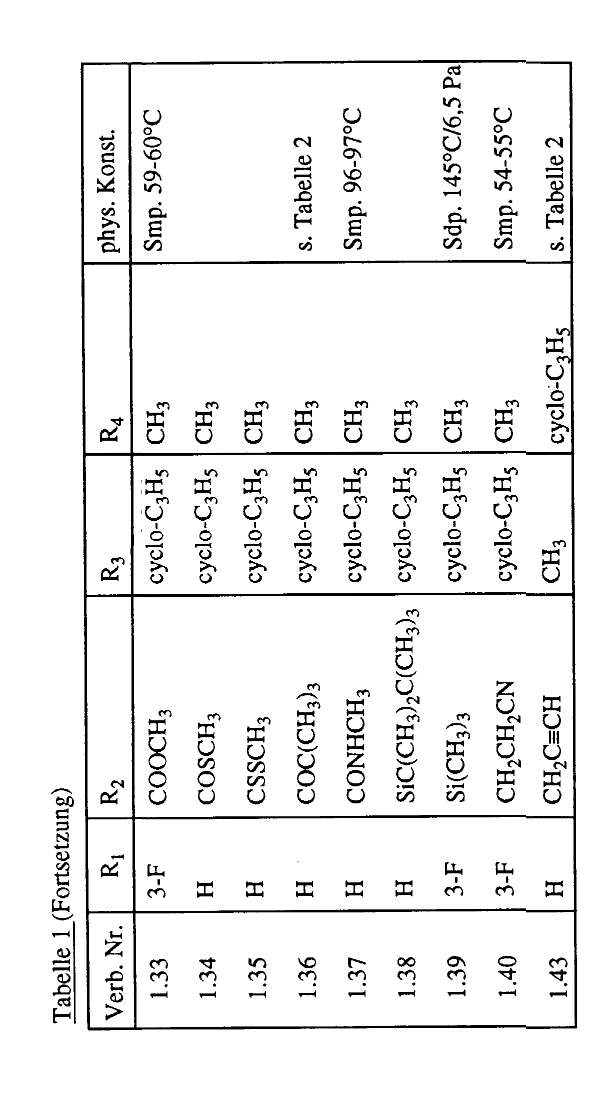

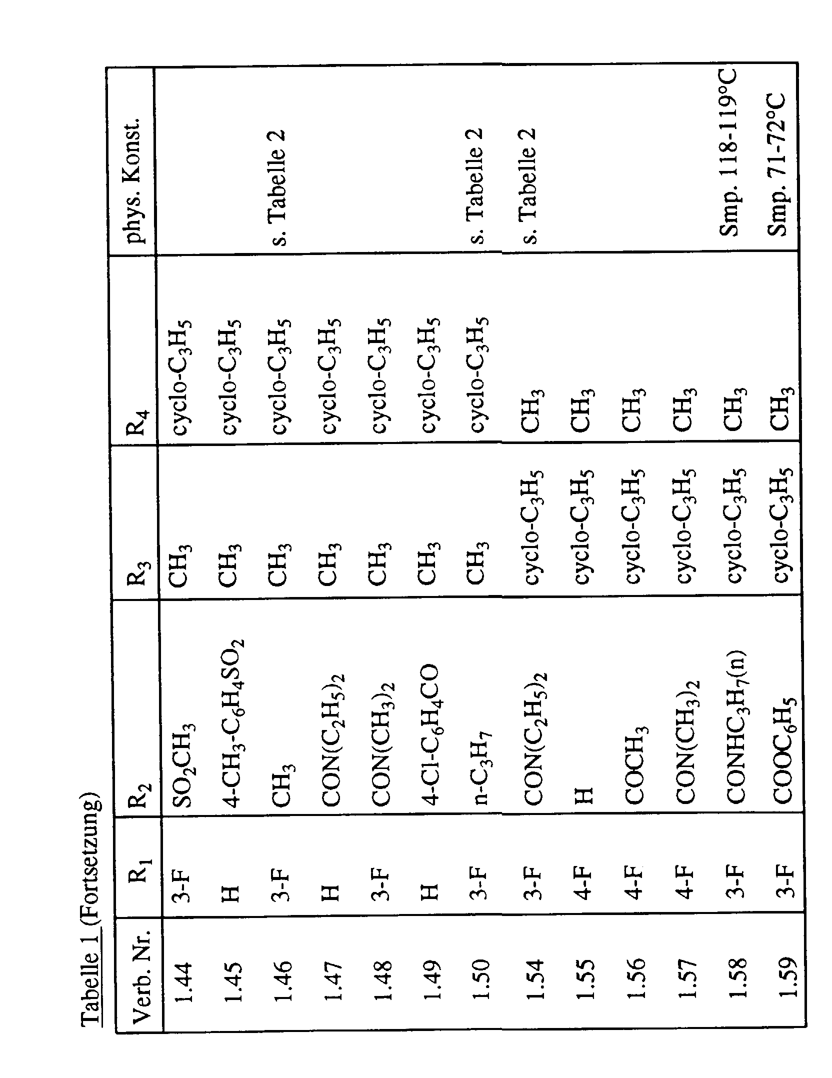

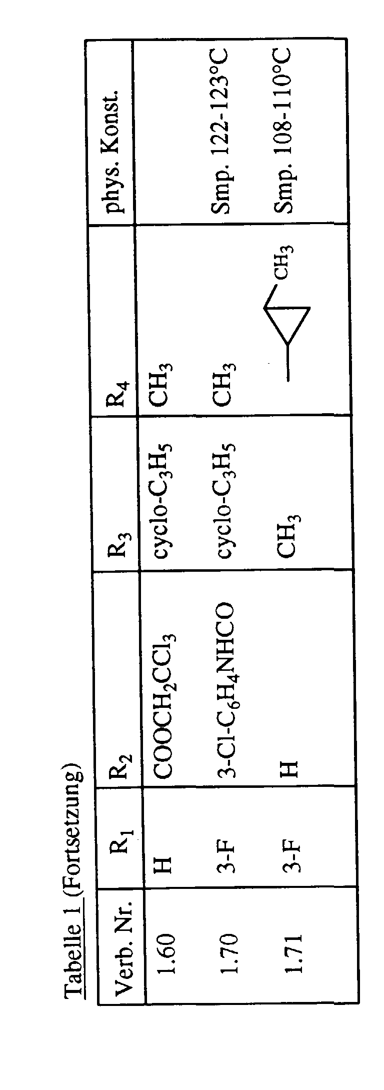

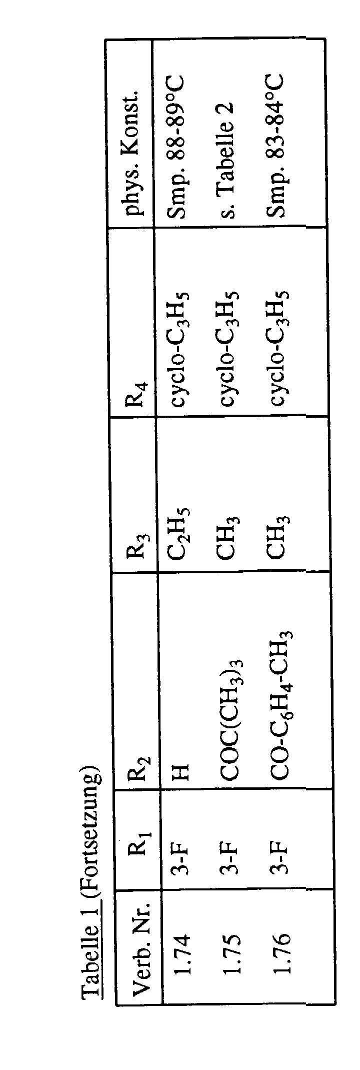

**Tabelle 2:**

| NMR-Daten nicht-kristallisierender Verbindungen | |
|---|---|
| Verb. Nr. | NMR; ppm-Werte; |
| 1.17 | 0,8-1,1 M (-CH₂CH₂-); 1,5-1,9 M (CH); 2,38 S (C-CH₃); 3,90 S (OCH₃); 6,53 S (H₅-Pyrim.); 7,1-7,8 M (arom. H) |
| 1.22 | 1,1-1,5 T (CH₃CH₂); 2,40 S (CH₃, Pyrim.); 2,6 Q (CH₂CH₃); 3,43 S (OCH₃); 4,38 S (CH₂O); 6,87 S (H₅-Pyrim.) |
| 1.26 | 0,8-1,1 M (CH₂CH₂); 1,7-2,0 M (CH); 2,25 S (CH₃CO); 2,32 S (CH₃-Pyrim.); 6,60 S (H₅-Pyrim.) |
| 1.36 | 0,9-1,2 M (CH₂CH₂); 1,37 S (C(CH₃)₃); 1,5-1,9 M (CH); 2,27 S (CH₃); 6,52 S (H₅-Pyrim.) |
| 1.43 | 0,8-1,1 M (CH₂CH₂); 1,6-2,0 M (CH); 2,37 (CH₃); 2,45 T (HC≡C-); 4,80 D (CH₂C≡C); 6,60 S (H₅-Pyrim.) |
| 1.46 | 0,8-1,1 M (CH₂CH₂); 1,5-2,0 M (CH); 2,40 S (CH₃-Pyrim.); 3,92 S (OCH₃); 6,60 S (H₅-Pyrim.) |
| 1.50 | 1,03 T+M (CH₃, CH₂CH₂); 1,5-2,0 M (CH₂, CH); 2,40 S (CH₃-Pyrim.), 4,03 T (OCH₂); 6,60 S (H₅Pyrim.) |
| 1.54 | 0,7-1,3 T+M (CH₃, CH₂CH₂); 1,5-2,0 M (CH); 2,33 S (CH₃); 3,47 Q (CH₂CH₃); 6,60 S (H₅-Pyrim.) |
| 1.75 | 0,7-1,2 M (CH₂CH₂); 1,4 S (C(CH₃)₃); 2,32 S (CH₃); 6,68 S (H₅-Pyrim); 7,2-7,6 M (arom. H). |

### 2. Formulierungsbeispiele für flüssige Wirkstoffe der Formel I (% = Gewichtsprozent)

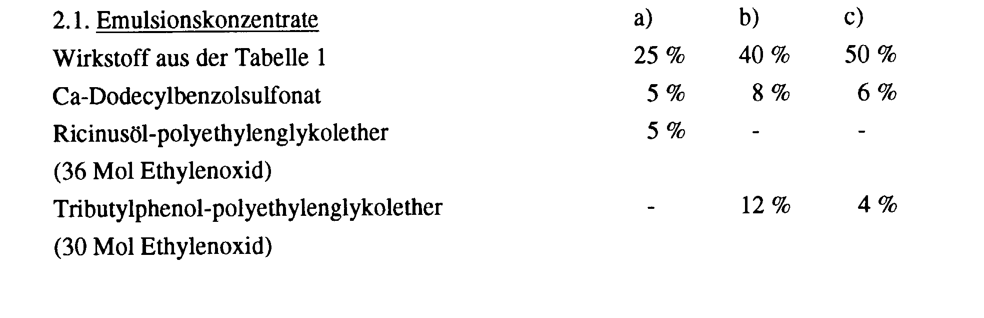

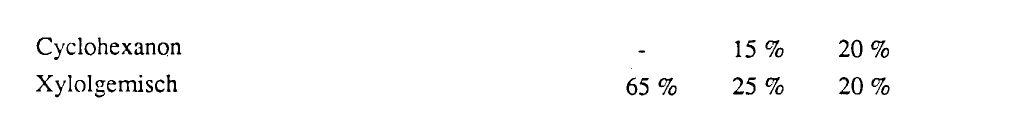

Aus solchen Konzentraten können durch Verdünnen mit Wasser Emulsionen jeder gewünschten Konzentration hergestellt werden.

| 2.2. Lösungen | a) | b) | c) | d) |
|---|---|---|---|---|
| Wirkstoff aus der Tabelle 1 | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |
| (MG = Molekulargewicht) | | | | |

Die Lösungen sind zur Anwendung in Form kleinster Tropfen geeignet.

| 2.3 Granulate | a) | b) |
|---|---|---|
| Wirkstoff aus der Tabelle 1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufbesprüht und das Lösungsmittel anschliessend im Vakuum abgedampft.

| 2.4. Stäubemittel | a) | b) |
|---|---|---|
| Wirkstoff aus der Tabelle 1 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

### Formulierungsbeispiele für feste Wirkstoffe der Formel I (% = Gewichtsprozent)

| 2.5 Spritzpulver | a) | b) | c) |
|---|---|---|---|
| Wirkstoff aus der Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.6 Emulsions-Konzentrat | |
|---|---|
| Wirkstoff aus der Tabelle 1 | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 34 % |
| Xylolgemisch | 50 % |

Aus diesem Konzentrat können durch Verdünnen mit Wasser Emulsionen jeder gewünschten Konzentration hergestellt werden.

| 2.7 Stäubemittel | a) | b) |
|---|---|---|
| Wirkstoff aus der Tabelle 1 | 5 % | 8 % |
| Talkum | 95 % | - |
| Kaolin | - | 92 % |

Man erhält anwendungsfertige Stäubemittel, indem der Wirkstoff mit dem Träger vermischt auf einer geeigneten Mühle vermahlen wird.

| 2.8 Extruder Granulat | |
|---|---|
| Wirkstoff aus der Tabelle 1 | 10 % |
| Na-Ligninsulfonat | 2 % |
| Carboxylmethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.9 Umhüllungs-Granulat | |
|---|---|
| Wirkstoff aus der Tabelle 1 | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| 2.10 Suspensions-Konzentrat | |
|---|---|
| Wirkstoff aus der Tabelle 1 | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

### 3. Biologische Beispiele

### Beispiel 3.1: Wirkung gegen Venturia inaequalis auf Apfeltrieben Residual-protektive Wirkung

Apfelstecklinge mit 10-20 cm langen Frischtrieben werden mit einer aus Spritzpulver des Wirkstoffes hergestellten Spritzbrühe (0,006 % Aktivsubstanz) besprüht. Nach 24 Stunden werden die behandelten Pflanzen mit einer Konidiensuspension des Pilzes infiziert. Die Pflanzen werden dann während 5 Tagen bei 90-100 % relativer Luftfeuchtigkeit inkubiert und während 10 weiteren Tagen in einem Gewächshaus bei 20-24°C aufgestellt Der Schorfbefall wird 15 Tage nach der Infektion beurteilt.

Verbindungen aus Tabelle 1 zeigen gegen Venturia gute Wirksamkeit (Befall: weniger als 20 %). So reduzierten z.B. die Verbindungen Nr. 1.13, 1.16, 1.17, 1.26, 1.36 und 1.37 den Venturia-Befall auf 0 bis 10 %. Unbehandelte aber infizierte Kontrollpflanzen wiesen dagegen einen Venturia-Befall von 100 % auf.

### Beispiel 3.2: Wirkung gegen Botrytis cinerea an Apfelfrüchten Residual-protektive Wirkung

Künstlich verletzte Aepfel werden behandelt, indem eine aus Spritzpulver der Wirksubstanz hergestellte Spritzbrühe (0,002 % Aktivsubstanz) auf die Verletzungsstellen aufgetropft wird. Die behandelten Früchte werden anschliessend mit einer Sporensuspension des Pilzes inokuliert und während einer Woche bei hoher Luftfeuchtigkeit und ca. 20°C inkubiert. Bei der Auswertung werden die angefaulten Verletzungsstellen gezählt und daraus die fungizide Wirkung der Testsubstanz abgeleitet.

Verbindungen aus Tabelle 1 zeigen gegen Botrytis gute Wirksamkeit (Befall: weniger als 20 %). So reduzierten z.B. die Verbindungen Nr. 1.13, 1.16, 1.17, 1.22, 1.36, 1.37 und 1.54 den Botrytis-Befall auf 0 bis 10 %. Unbehandelte aber infizierte Kontrollpflanzen wiesen dagegen einen Botrytis-Befall von 100 % auf.

### Beispiel 3.3: Wirkung gegen Erysiphe graminis auf Gerste

### a) Residual-protektive Wirkung

Ca. 8 cm hohe Gerstenpflanzen werden mit einer aus Spritzpulver des Wirkstoffes hergestellten Spritzbrühe (0,006 % Aktivsubstanz) besprüht. Nach 3-4 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

Verbindungen aus Tabelle 1 zeigen gegen Erysiphe gute Wirksamkeit (Befall: weniger als 20 %). So reduzierten z.B. die Verbindungen Nr. 1.16, 1.26 und 1.36 den Erysiphae-Befall auf 0 bis 10 %. Unbehandelte aber infizierte Kontrollpflanzen wiesen dagegen einen Erysiphae-Befall von 100 % auf.

## Patentansprüche (Patentansprüche für folgende(n) Vertragsstaat(en): CH, DE, FR, GB, IT, LI, LU, NL)

1. Verbindungen der Formel I 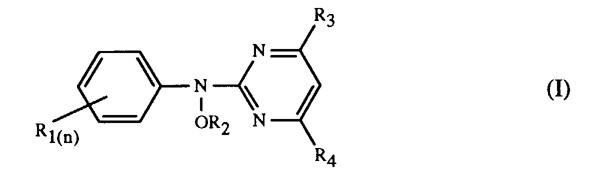 in welcher bedeuten:
R₁ Wasserstoff, Halogen, Methyl, Methoxy oder Trifluormethyl;
n 1 oder 2;
R₂ Wasserstoff, C₁-C₆-Alkyl, mit Halogen, Cyano oder Methoxy substituiertes C₁-C₃-Alkyl, oder C₂-C₅-Alkenyl, C₂-C₅-Alkinyl, Benzyl, COR₅, CON(R₅)R₆,
CSN(R₅)R₆, CO(OR₆), CO(SR₆), CS(SR₅), SO₂R₇, PO(OR₆)₂ oder Si(R₆)₃;
R₃ und R₄ unabhängig voneinander Wasserstoff, Cyclopropyl oder Methylcyclopropyl und einer von R₃ und R₄ kann auch C₁-C₅-Alkyl sein;
R₅ Wasserstoff, C₁-C₆-Alkyl, mit 1 bis 3 Halogen substituiertes C₁-C₃-Alkyl, mit OR₆ oder SR₆ substituiertes C₁-C₃-Alkyl, C₂-C₅-Mkenyl, mit 1 bis 3 Halogen substituiertes C₂-C₅-Alkenyl, C₂-C₅-Alkinyl, C₃-C₆-Cycloafkyl, Phenyl oder 1- bis 3-fach mit Halogen, C₁-C₃-Alkyl, C₁-C₃-Halogenalkyl, C₁-C₃-Alkoxy, Nitro oder Cyano substituiertes Phenyl;
R₆ C₁-C₆-Alkyl;
R₇ C₁-C₃-Alkyl, Phenyl, mit Halogen, C₁-C₂-Alkyl, C₁-C₂-Halogenalkyl, Methoxy, Nitro, 1- oder 2-fach substituiertes Phenyl; einschliesslich der Säureadditionssalze der Verbindungen der Formel I.

2. Verbindungen gemäss Anspruch 1 der Formel I, in welcher bedeuten:
R₁ Wasserstoff, Fluor, Chlor oder Brom;
n 1;
R₂ Wasserstoff, C₁-C₄-Mkyl, C₂-C₃-Alkenyl, C₂-C₃-Alkinyl, COR₅, CON(R₅)R₆, CSNR₅(R₆) oder COOR₆;
R₃ und R₄ unabhängig voneinander Wasserstoff, Cyclopropyl oder Methylcyclopropyl und einer von R₃ und R₄ kann auch C₁-C₅-Alkyl sein;
R₅ Wasserstoff, C₁-C₆-Mkyl, C₃-C₆-Cycloalkyl, C₂-C₅-Alkenyl, C₂-C₅-AIkinyl, Phenyl oder einfach mit Fluor, Chlor, Brom, Methyl, Trifluormethyl, Trichlormethyl,
Tribrommethyl, Methoxy, Nitro oder Cyano substituiertes Phenyl; R₆ C₁-C₃-Alkyl.

3. Verbindungen gemäss Anspruch 1 der Formel I, in welcher bedeuten:
R₁ Wasserstoff, Fluor oder Chlor;
n 1;
R₂ Wasserstoff, C₁-C₃-Alkyl, COR₅, CON(R₅)R₆, CSNR₅(R₆) oder COOR₆;
R₃ und R₄ unabhängig voneinander Cyclopropyl oder Methylcyclopropyl und einer von R₃ und R₄ kann auch C₁-C₃-Alkyl sein;
R₅ Wasserstoff oder C₁-C₄-Alkyl;
R₆ C₁-C₃-Alkyl.

4. Eine Verbindung der Formel I gemäss Anspruch 1 ausgewählt aus der Gruppe:
N-(4-Methyl-6-cyclopropylpyrimidinyl-2)-phenylhydroxylamin;
N-(4-Methyl-6-cyclopropylpyrimidinyl-2)-3-fluorphenylhydroxylamin;
O-Acetyl-N-(4-methyl-6-cyclopropylpyrimidinyl-2)-phenylhydroxylamin;
O-Pivaloyl-N-(4-methyl-6-cyclopropylpyrimidinyl-2)-phenylhydroxylamin.

5. Verfahren zur Herstellung von Verbindungen der Formel I, dadurch gekennzeichnet, dass man umsetzt:
A) ein Pyrimidinylhalogenid der Formel II 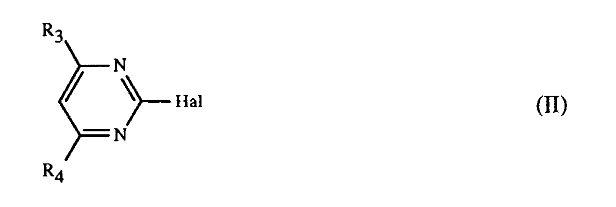 mit einem Phenylhydroxylamin der Formel III 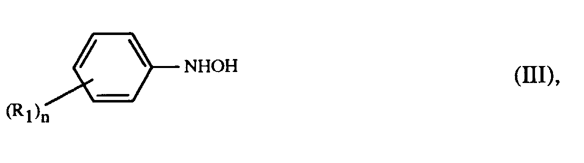 worin R₁, R₃ und R₄ und n die unter Formel I angegebenen Bedeutungen besitzen und Hal ein Halogen, vorzugsweise Chlor oder Brom, darstellt, in Gegenwart einer Säure in inerten organischen Lösungsmitteln bei Temperaturen von -30° bis 100°C, bevorzugt 0° bis 50°C; und
B) die daraus resultierende Verbindung Ia 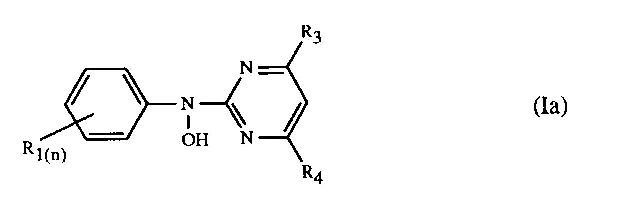
C) mit einer Verbindung der Formel IV
R₂ - X (IV),
worin R₁, R₂, R₃, R₄ und n die unter Formel I angegebenen Bedeutungen besitzen und X Halogen, vorzugsweise Chlor oder Brom, oder den Imidazolylrest darstellt; oder
D) mit einem Anhydrid der Formel V
(R₅CO)₂O (V);
oder
E) mit einem Isocyanat der Formel VI
R₅-N=C=Y (VI);
worin Y Sauerstoff oder Schwefel darstellt, in inerten organischen Lösungsmitteln bei Temperaturen von -20° bis 150°C und bezüglich der Reaktionen (C) und (D) in Gegenwart eines säurebindenen Mittels.

6. Mittel zur Bekämpfung oder Verhütung eines Befalls durch schädliche Mikroorganismen, dadurch gekennzeichnet, dass es als aktive Komponente mindestens eine Verbindung der Formel I gemäss Anspruch 1 enthält, zusammen mit einem geeigneten Trägermaterial.

7. Mittel gemäss Anspruch 6, dadurch gekennzeichnet, dass es als aktive Komponente mindestens eine Verbindung der Formel I gemäss Anspruch 2 enthält.

8. Mittel gemäss Anspruch 6, dadurch gekennzeichnet, dass es als aktive Komponente mindestens eine Verbindung der Formel I gemäss Anspruch 3 enthält.

9. Mittel gemäss Anspruch 6, dadurch gekennzeichnet, dass es als aktive Komponente mindestens eine Verbindung der Formel I gemäss Anspruch 4 enthält.

10. Verfahren zur Bekämpfung oder Verhütung eines Befalls von Kulturpflanzen durch phytopathogene Mikroorganismen, dadurch gekennzeichnet, dass man als Wirkstoff eine Verbindung der Formel I gemäss Anspruch 1 auf die Pflanze, auf Pflanzenteile oder ihren Standort appliziert.

11. Verfahren gemäss Anspruch 10, dadurch gekennzeichnet, dass man als Wirkstoff eine Verbindung gemäss einem der Ansprüche 2 bis 4 appliziert.

12. Verfahren gemäss Anspruch 10, dadurch gekennzeichnet, dass phytopathogene Fungi bekämpft werden.

13. Verfahren zur Herstellung eines agrochemischen Mittels gemäss Anspruch 6, dadurch gekennzeichnet, dass man mindestens eine Verbindung der Formel I gemäss Anspruch 1 mit geeigneten festen oder flüssigen Zusatzstoffen und/oder Tensiden innig vermischt.

## Patentansprüche (Patentansprüche für folgende(n) Vertragsstaat(en): ES)

1. Verfahren zur Herstellung von Verbindungen der Formel I 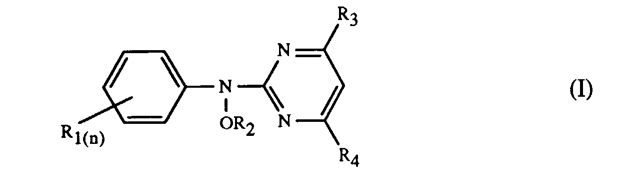 in welcher bedeuten:
R₁ Wasserstoff, Halogen, Methyl, Methoxy oder Trifluormethyl;
n 1 oder 2;
R₂ Wasserstoff, C₁-C₆-Alkyl, mit Halogen, Cyano oder Methoxy substituiertes
C₁-C₃-Alkyl, oder C₂-C₅-Alkenyl, C₂-C₅-Alkinyl, Benzyl, COR₅, CON(R₅)R₆, CSN(R₅)R₆, CO(OR₆), CO(SR₆), CS(SR₅), SO₂R₇, PO(OR₆)₂ oder Si(R₆)₃;
R₃ und R₄ unabhängig voneinander Wasserstoff, Cyclopropyl oder Methylcyclopropyl und einer von R₃ und R₄ kann auch C₁-C₅-Alkyl sein;
R₅ Wasserstoff, C₁-C₆-Alkyl, mit 1 bis 3 Halogen substituiertes C₁-C₃-Alkyl, mit OR₆ oder SR₆ substituiertes C₁-C₃-Alkyl, C₂-C₅-Alkenyl, mit 1 bis 3 Halogen substituiertes C₂-C₅-Alkenyl, C₂-C₅-Alkinyl, C₃-C₆-Cycloalkyl, Phenyl oder 1- bis 3-fach mit Halogen, C₁-C₃-Alkyl, C₁-C₃-Halogenalkyl, C₁-C₃-Alkoxy, Nitro oder Cyano substituiertes Phenyl;
R₆ C₁-C₆-Alkyl;
R₇ C₁-C₃-Alkyl, Phenyl, mit Halogen, C₁-C₂-Alkyl, C₁-C₂-Halogenalkyl, Methoxy, Nitro, 1- oder 2-fach substituiertes Phenyl; einschliesslich der Säureadditionssalze der Verbindungen der Formel I, dadurch gekennzeichnet, dass man umsetzt:
A) ein Pyrimidinylhalogenid der Formel II 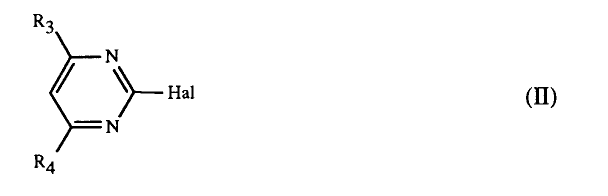 mit einem Phenylhydroxylamin der Formel III 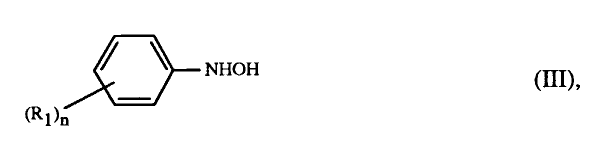 worin R₁, R₃ und R₄ und n die unter Formel I angegebenen Bedeutungen besitzen und Hal ein Halogen, vorzugsweise Chlor oder Brom, darstellt, in Gegenwart einer Säure in inerten organischen Lösungsmitteln bei Temperaturen von -30° bis 100°C, bevorzugt 0° bis 50°C; und
B) die daraus resultierende Verbindung Ia 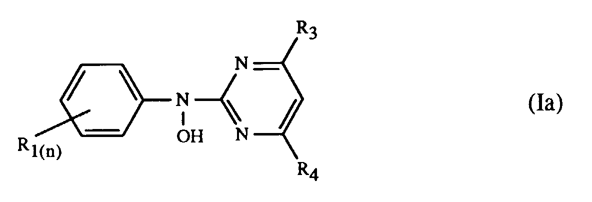
C) mit einer Verbindung der Formel IV
R₂ - X (IV),
worin R₁, R₂, R₃, R₄ und n die unter Formel I angegebenen Bedeutungen besitzen und X Halogen, vorzugsweise Chlor oder Brom, oder den Imidazolylrest darstellt; oder
D) mit einem Anhydrid der Formel V
(R₅CO)₂O (V);
oder
E) mit einem Isocyanat der Formel VI
R₅-N=C=Y (VI);
worin Y Sauerstoff oder Schwefel darstellt, in inerten organischen Lösungsmitteln bei Temperaturen von -20° bis 150°C und bezüglich der Reaktionen (C) und (D) in Gegenwart eines säurebindenen Mittels.

2. Verfahren gemäss Anspruch 1, dadurch gekennzeichnet, dass man Verbindungen der Formel I herstellt, in welcher bedeuten:
R₁ Wasserstoff, Fluor, Chlor oder Brom;
n 1;
R₂ Wasserstoff, C₁-C₄-Alkyl, C₂-C₃-Alkenyl, C₂-C₃-Alkinyl, COR₅, CON(R₅)R₆, CSNR₅(R₆) oder COOR₆;
R₃ und R₄ unabhängig voneinander Wasserstoff, Cyclopropyl oder Methylcyclopropyl und einer von R₃ und R₄ kann auch C₁-C₅-Alkyl sein;
R₅ Wasserstoff, C₁-C₆-Alkyl, C₃-C₆-Cycloalkyl, C₂-C₅-Alkenyl, C₂-C₅-Alkinyl, Phenyl oder einfach mit Fluor, Chlor, Brom, Methyl, Trifluormethyl, Trichlormethyl, Tribrommethyl, Methoxy, Nitro oder Cyano substituiertes Phenyl;
R₆ C₁-C₃-Alkyl.

3. Verfahren gemäss Anspruch 1, dadurch gekennzeichnet, dass man Verbindungen der Formel I herstellt, in welcher bedeuten:
R₁ Wasserstoff, Fluor oder Chlor;
n 1;
R₂ Wasserstoff, C₁-C₃-Alkyl, COR₅, CON(R₅)R₆, CSNR₅(R₆) oder COOR₆;
R₃ und R₄ unabhängig voneinander Cyclopropyl oder Methylcyclopropyl und einer von R₃ und R₄ kann auch C₁-C₃-Alkyl sein;
R₅ Wasserstoff oder C₁-C₄-Alkyl;
R₆ C₁-C₃-Alkyl.

4. Verfahren gemäss Anspruch 1, dadurch gekennzeichnet, dass man folgende Verbindungen der Formel I herstellt:
N-(4-Methyl-6-cyclopropylpyrimidinyl-2)-phenylhydroxylamin;
N-(4-Methyl-6-cyclopropylpyrimidinyl-2)-3-fluorphenylhydroxylamin;
O-Acetyl-N-(4-methyl-6-cyclopropylpyrimidinyl-2)-phenylhydroxylamin;
O-Pivaloyl-N-(4-methyl-6-cyclopropylpyrimidinyl-2)-phenylhydroxylamin.

5. Mittel zur Bekämpfung oder Verhütung eines Befalls durch schädliche Mikroorganismen, dadurch gekennzeichnet, dass es als aktive Komponente mindestens eine Verbindung der Formel I gemäss Anspruch 1 enthält, zusammen mit einem geeigneten Trägermaterial.

6. Mittel gemäss Anspruch 5, dadurch gekennzeichnet, dass es als aktive Komponente mindestens eine Verbindung der Formel I gemäss Anspruch 2 enthält.

7. Mittel gemäss Anspruch 5, dadurch gekennzeichnet, dass es als aktive Komponente mindestens eine Verbindung der Formel I gemäss Anspruch 3 enthält.

8. Mittel gemäss Anspruch 5, dadurch gekennzeichnet, dass es als aktive Komponente mindestens eine Verbindung der Formel I gemäss Anspruch 4 enthält.

## Claims (Claims for the following Contracting State(s): CH, DE, FR, GB, IT, LI, LU, NL)

1. A compound of the formula I 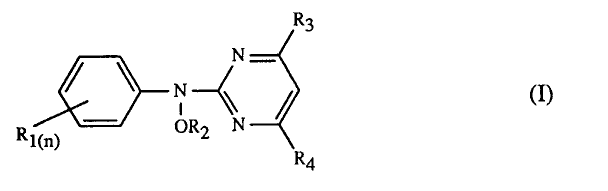 in which
R₁ is hydrogen, halogen, methyl, methoxy or trifluoromethyl; n is 1 or 2;
R₂ is hydrogen, C₁-C₆alkyl, C₁-C₃alkyl which is substituted by halogen, cyano or methoxy, or is C₂-C₅alkenyl, C₂-C₅alkynyl, benzyl, COR₅, CON(R₅)R₆, CSN(R₅)R₆, CO(OR₆), CO(SR₆), CS(SR₅), SO₂R₇, PO(OR₆)₂ or Si(R₆)₃;
R₃ and R₄ independently of one another are hydrogen, cyclopropyl or methylcyclopropyl and one of R₃ and R₄ can also be C₁-C₅alkyl;
R₅ is hydrogen, C₁-C₆alkyl, C₁-C₃alkyl which is substituted by 1 to 3 halogens, C₁-C₃alkyl which is substituted by OR₆ or SR₆, or is C₂-C₅alkenyl, C₂-C₅alkenyl which is substituted by 1 to 3 halogens, C₂-C₅alkynyl, C₃-C₆cycloalkyl, phenyl, or phenyl which is monosubstituted to trisubstituted by halogen, C₁-C₃alkyl, C₁-C₃haloalkyl, C₁-C₃alkoxy, nitro or cyano;
R₆ is C₁-C₆alkyl;
R₇ is C₁-C₃alkyl, phenyl, phenyl which is monosubstituted or disubstituted by halogen, C₁-C₂alkyl, C₁-C₂haloalkyl, methoxy or nitro; including the acid addition salts of the compounds of the formula I.

2. A compound according to claim 1, of the formula I, in which
R₁ is hydrogen, fluorine, chlorine or bromine;
n is 1;
R₂ is hydrogen, C₁-C₄alkyl, C₂-C₃alkenyl, C₂-C₃alkynyl, COR₅, CON(R₅)R₆, CSNR₅(R₆) or COOR₆;
R₃ and R₄ independently of one another are hydrogen, cyclopropyl or methylcyclopropyl and one of R₃ and R₄ can also be C₁-C₅alkyl;
R₅ is hydrogen, C₁-C₆alkyl, C₃-C₆cycloalkyl, C₂-C₅alkenyl, C₂-C₅alkynyl, phenyl, or phenyl which is monosubstituted by fluorine, chlorine, bromine, methyl, trifluoromethyl, trichloromethyl, tribromomethyl, methoxy, nitro or cyano;
and R₆ is C₁-C₃alkyl.

3. A compound according to claim 1, of the formula I, in which
R₁ is hydrogen, fluorine or chlorine;
nis 1;
R₂ is hydrogen, C₁-C₃alkyl, COR₅, CON(R₅)R₆, CSNR₅(R₆) or COOR₆;
R₃ and R₄ independently of one another are cyclopropyl or methylcyclopropyl and one of R₃ and R₄ can also be C₁-C₃alkyl;
R₅ is hydrogen or C₁-C₄alkyl; .
and R₆ is C₁-C₃alkyl.

4. A compound of the formula I, according to claim 1, selected from the group:
N-(4-methyl-6-cyclopropylpyrimidin-2-yl)-phenylhydroxylamine;
N-(4-methyl-6-cyclopropylpyrimidin-2-yl)-3-fluorophenylhydroxylamine;
O-acetyl-N-(4-methyl-6-cyclopropylpyrimidin-2-yl)-phenylhydroxylamine;
O-pivaloyl-N-(4-methyl-6-cyclopropylpyrimidin-2-yl)-phenylhydroxylamine.

5. A process for the preparation of compounds of the formula I, which comprises reacting:
A) a pyrimidinyl halide of the formula II 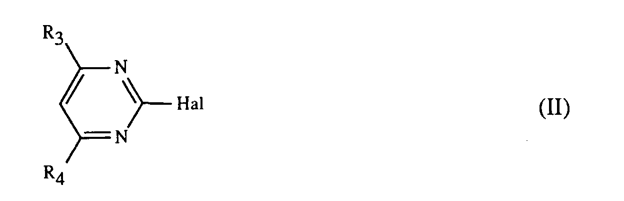 with a phenylhydroxylamine of the formula III 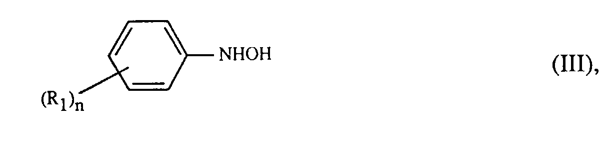 in which R₁, R₃ and R₄ and n are as defined under formula I and Hal is a halogen, preferably chlorine or bromine, in the presence of an acid in inert organic solvents, at temperatures from -30° to 100°C, preferably 0° to 50°C; and
B) the resulting compound Ia 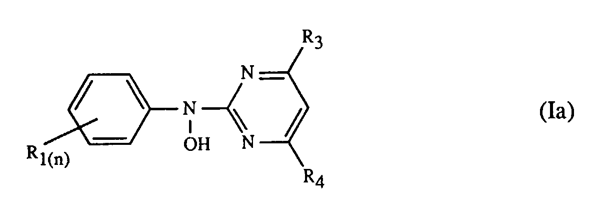
C) with a compound of the formula IV
R₂ - X (IV),
in which R₁, R₂, R₃, R₄ and n are as defined under formula I and X is halogen, preferably chlorine or bromine, or the imidazolyl radical; or
D) with an anhydride of the formula V
(R₅CO)₂O (V);
or
E) with an isocyanate of the formula VI
R₅-N=C=Y (VI),
in which Y is oxygen or sulfur, in inert organic solvents, at temperatures from -20° to 150°C, and, as regards reactions (C) and (D), in the presence of an acid-binding agent.

6. A composition for controlling or preventing infestation by harmful microorganisms, which comprises, as the active component, at least one compound of the formula I according to claim 1, together with a suitable carrier material.

7. A composition according to claim 6, which comprises, as the active component, at least one compound of the formula I according to claim 2.

8. A composition according to claim 6, which comprises, as the active component, at least one compound of the formula I according to claim 3.

9. A composition according to claim 6, which comprises, as the active component, at least one compound of the formula I according to claim 4.

10. A method of controlling or preventing infestation of crop plants by phytopathogenic microorganisms, which comprises applying, as the active substance, a compound of the formula I according to claim 1 to the plant, parts of the plant or the site where it grows.

11. A method according to claim 10, in which a compound according to any one of claims 2 to 4 is applied as the active substance.

12. A method according to claim 10, in which phytopathogenic fungi are controlled.

13. A process for the preparation of an agrochemical composition according to claim 6, which comprises intimately mixing at least one compound of the formula I according to claim 1 with suitable solid or liquid additives and/or surfactants.

## Claims (Claims for the following Contracting State(s): ES)

1. A process for the preparation of compounds of the formula I 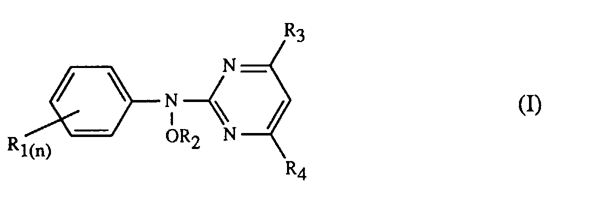 in which
R₁ is hydrogen, halogen, methyl, methoxy or trifluoromethyl;
n is 1 or 2;
R₂ is hydrogen, C₁-C₆alkyl, C₁-C₃alkyl which is substituted by halogen, cyano or methoxy, or is C₂-C₅alkenyl, C₂-C₅alkynyl, benzyl, COR₅, CON(R₅)R₆, CSN(R₅)R₆, CO(OR₆), CO(SR₆), CS(SR₅), SO₂R₇, PO(OR₆)₂ or Si(R₆)₃;
R₃ and R₄ independently of one another are hydrogen, cyclopropyl or methylcyclopropyl and one of R₃ and R₄ can also be C₁-C₅alkyl;
R₅ is hydrogen, C₁-C₆alkyl, C₁-C₃alkyl which is substituted by 1 to 3 halogens, C₁-C₃alkyl which is substituted by OR₆ or SR₆, or is C₂-C₅alkenyl, C₂-C₅alkenyl which is substituted by 1 to 3 halogens, C₂-C₅alkynyl, C₃-C₆cycloalkyl, phenyl, or phenyl which is monosubstituted to trisubstituted by halogen, C₁-C₃alkyl, C₁-C₃haloalkyl, C₁-C₃alkoxy, nitro or cyano;
R₆ is C₁-C₆alkyl;
R₇ is C₁-C₃alkyl, phenyl, phenyl which is monosubstituted or disubstituted by halogen, C₁-C₂alkyl, C₁-C₂haloalkyl, methoxy or nitro; including the acid addition salts of the compounds of the formula I, which comprises reacting:
A) a pyrimidinyl halide of the formula II 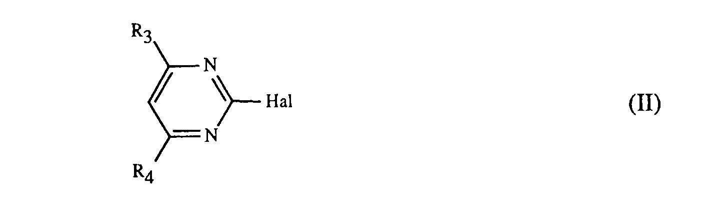 with a phenylhydroxylamine of the formula III 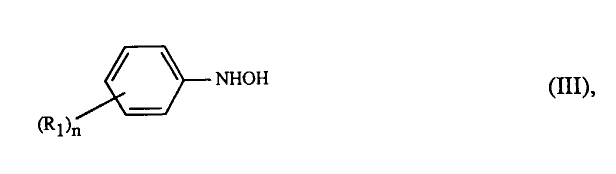 in which R₁, R₃ and R₄ and n are as defined under formula I and Hal is a halogen, preferably chlorine or bromine, in the presence of an acid in inert organic solvents, at temperatures from -30° to 100°C, preferably 0° to 50°C; and
B) the resulting compound Ia 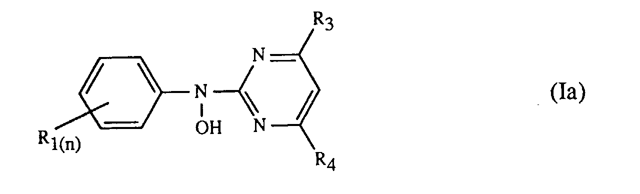
C) with a compound of the formula IV
R₂ - X (IV),
in which R₁, R₂, R₃, R₄ and n are as defined under formula I and X is halogen, preferably chlorine or bromine, or the imidazolyl radical; or
D) with an anhydride of the formula V
(R₅CO)₂O (V);
or
E) with an isocyanate of the formula VI
R₅-N=C=Y (VI),
in which Y is oxygen or sulfur, in inert organic solvents, at temperatures from -20° to 150°C, and, as regards reactions (C) and (D), in the presence of an acid-binding agent.

2. A process according to claim 1, which comprises preparing compounds of the formula I in which
R₁ is hydrogen, fluorine, chlorine or bromine;
nis 1;
R₂ is hydrogen, C₁-C₄alkyl, C₂-C₃alkenyl, C₂-C₃alkynyl, COR₅, CON(R₅)R₆, CSNR₅(R₆) or COOR₆;
R₃ and R₄ independently of one another are hydrogen, cyclopropyl or methylcyclopropyl and one of R₃ and R₄ can also be C₁-C₅alkyl;
R₅ is hydrogen, C₁-C₆alkyl, C₃-C₆cycloalkyl, C₂-C₅alkenyl, C₂-C₅alkynyl, phenyl, or phenyl which is monosubstituted by fluorine, chlorine, bromine, methyl, trifluoromethyl, trichloromethyl, tribromomethyl, methoxy, nitro or cyano;
and R₆ is C₁-C₃alkyl.

3. A process according to claim 1, which comprises preparing compounds of the formula I in which
R₁ is hydrogen, fluorine or chlorine;
n is 1;
R₂ is hydrogen, C₁-C₃alkyl, COR₅, CON(R₅)R₆, CSNR₅(R₆) or COOR₆;
R₃ and R₄ independently of one another are cyclopropyl or methylcyclopropyl and one of R₃ and R₄ can also be C₁-C₃alkyl;
R₅ is hydrogen or C₁-C₄alkyl;
and R₆ is C₁-C₃alkyl.

4. A process according to claim 1, which comprises preparing the following compounds of the formula I:
N-(4-methyl-6-cyclopropylpyrimidin-2-yl)-phenylhydroxylamine;
N-(4-methyl-6-cyclopropylpyrimidin-2-yl)-3-fluorophenylhydroxylamine;
O-acetyl-N-(4-methyl-6-cyclopropylpyrimidin-2-yl)-phenylhydroxylamine;
O-pivaloyl-N-(4-methyl-6-cyclopropylpyrimidin-2-yl)-phenylhydroxylamine.

5. A composition for controlling or preventing infestation by harmful microorganisms, which comprises, as the active component, at least one compound of the formula I according to claim 1, together with a suitable carrier material.

6. A composition according to claim 5, which comprises, as the active component, at least one compound of the formula I according to claim 2.

7. A composition according to claim 5, which comprises, as the active component, at least one compound of the formula I according to claim 3.

8. A composition according to claim 5, which comprises, as the active component, at least one compound of the formula I according to claim 4.

## Revendications (Revendications pour l'(les) Etat(s) contractant(s) suivant(s): CH, DE, FR, GB, IT, LI, LU, NL)

1. Composés de formule I 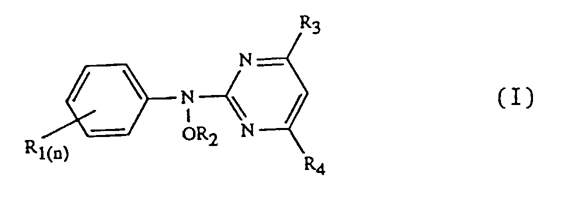 dans laquelle
R₁ représente l'hydrogène, un halogène, le méthyle, le méthoxy ou le trifluorométhyle;
n est égal à 1 ou 2;
R₂ représente l'hydrogène, un alkyle en C₁-C₆, un alkyle en C₁-C₃ substitué par un halogène, le cyano ou le méthoxy ou un alcényle en C₂-C₅, un alcynyle en C₂-C₅, le benzyle, COR₅, CON(R₅)R₆, CSN(R₅)R₆, CO(OR₆), CO(SR₆), CS(SR₅), SO₂R₇, PO(OR₆)₂ ou Si(R₆)₃;
R₃ et R₄ représentent, indépendamment l'un de l'autre, l'hydrogène, le cyclopropyle ou le méthylcyclopropyle et l'un des radicaux R₃ et R₄ peut être également un alkyle en C₁-C₅;
R₅ représente l'hydrogène, un alkyle en C₁-C₆, un alkyle en C₁-C₃ substitué par 1 à 3 halogènes, un alkyle en C₁-C₃ substitué par OR₆ ou SR₆, un alcényle en C₂-C₅, un alcényle en C₂-C₅ substitué par 1 à 3 halogènes, un alcynyle en C₂-C₅, un cycloalkyle en C₃-C₆, le phényle ou un phényle mono-, di- ou trisubstitué par un halogène, un alkyle en C₁-C₃, un halogénoalkyle en C₁-C₃, un alcoxy en C₁-C₃, le nitro ou le cyano;
R₆ représente un alkyle en C₁-C₆;
R₇ représente un alkyle en C₁-C₃, le phényle ou un phényle mono- ou disubstitué par un halogène, un alkyle en C₁-C₂, un halogénoalkyle en C₁-C₂, le méthoxy, le nitro; incluant les sels d'addition avec des acides des composés de formule I.

2. Composés de formule I selon la revendication 1, dans laquelle
R₁ représente l'hydrogène, le fluor, le chlore ou le brome;
n est égal à 1;
R₂ représente l'hydrogène, un alkyle en C₁-C₄, un alcényle en C₂-C₃, Un alcynyle en C₂-C₃, COR₅, CON(R₅)R₆, CSNR₅(R₆) ou COOR₆;
R₃ et R₄ représentent, indépendamment l'un de l'autre, l'hydrogène, le cyclopropyle ou le méthylcyclopropyle et l'un des radicaux R₃ et R₄ peut être également un alkyle en C₁-C₅;
R₅ représente l'hydrogène, un alkyle en C₁-C₆, un cycloalkyle en C₃-C₆, un alcényle en C₂-C₅, un alcynyle en C₂-C₅, le phényle ou un phényle monosubstitué par le fluor, le chlore, le brome, le méthyle, le trifluorométhyle, le trichlorométhyle, le tribromométhyle, le méthoxy, le nitro ou le cyano;
R₆ représente un alkyle en C₁-C₃.

3. Composés de formule I selon la revendication 1, dans laquelle
R₁ représente l'hydrogène, le fluor ou le chlore;
n est égal à 1;
R₂ représente l'hydrogène, un alkyle en C₁-C₃, COR₅, CON(R₅)R₆, CSNR₅(R₆) ou COOR₆;
R₃ et R₄ représentent, indépendamment l'un de l'autre, le cyclopropyle ou le méthylcyclopropyle et l'un des radicaux R₃ et R₄ peut être également un alkyle en C₁-C₃;
R₅ représente l'hydrogène ou un alkyle en C₁-C₄;
R₆ représente un alkyle en C₁-C₃.

4. Un composé de formule I selon la revendication 1, choisi dans le groupe constitué par :
la N-(4-méthyl-6-cyclopropylpyrimidinyl-2)-phénylhydroxylamine;
la N-(4-méthyl-6-cyclopropylpyrimidinyl-2)-3-fluorophénylhydroxylamine;
la O-acétyl-N-(4-méthyl-6-cyclopropylpyrimidinyl-2)-phénylhydroxylamine;
la O-pivaloyl-N-(4-méthyl-6-cyclopropylpyrimidinyl-2)-phénylhydroxylamine.

5. Procédé pour la préparation des composés de formule I, caractérisé en ce que l'on fait réagir :
A) un halogénure de pyrimidinyle de formule II 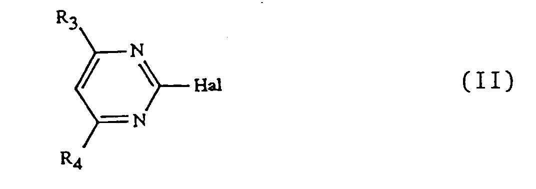 avec une phénylhydroxylamine de formule III 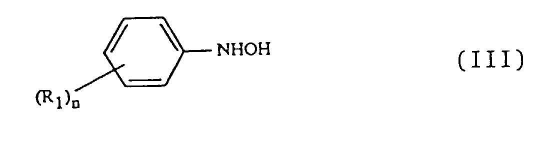 où R₁, R₃, R₄ et n ont les significations indiquées pour la formule I et Hal représente un halogène, de préférence le chlore ou le brome, en présence d'un acide dans des solvants organiques inertes à des températures allant de -30°C à 100°C, de préférence allant de 0°C à 50°C et
B) le composant la résultant 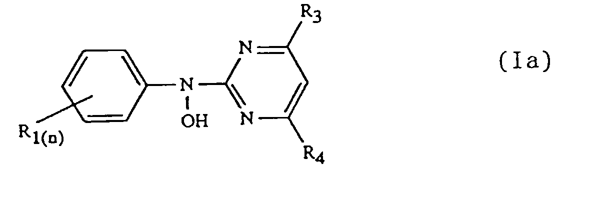
C) avec un composé de formule IV
R₂-X (IV),
où R₁, R₂, R₃, R₄ et n ont les significations indiquées pour la formule I et X représente un halogène, de préférence le chlore ou le brome ou le reste imidazolyle; ou
D) avec un anhydride de formule V
(R₅CO)₂O (V);
ou
E) avec un isocyanate de formule VI
R₅-N=C=Y (VI),
où Y représente l'oxygène ou le soufre, dans des solvants organiques inertes à des températures allant de -20°C à 150°C et, en ce qui concerne les réactions (C) et (D), en présence d'un accepteur d'acide.

6. Composition pour lutter contre ou prévenir une attaque par les microorganismes nuisibles, caractérisée en ce qu'elle contient, comme composant actif, au moins un composé de formule I selon la revendication 1, associé à un support approprié.

7. Composition selon la revendication 6, caractérisée en ce qu'elle contient, comme composant actif, au moins un composé de formule I selon la revendication 2.

8. Composition selon la revendication 6, caractérisée en ce qu'elle contient, comme composant actif, au moins un composé de formule I selon la revendication 3.

9. Composition selon la revendication 6, caractérisée en ce qu'elle contient, comme composant actif, au moins un composé de formule I selon la revendication 4.

10. Procédé pour lutter contre ou prévenir une attaque des plantes cultivées par les microorganismes phytopathogènes, caractérisé en ce que l'on applique, comme substance active, un composé de formule I selon la revendication 1 sur la plante, sur des parties de la plante ou sur l'endroit où elle se trouve.

11. Procédé selon la revendication 10, caractérisé en ce que l'on applique, comme substance active, un composé selon l'une des revendications 2 à 4.

12. Procédé selon la revendication 10, caractérisé en ce qu'on lutte contre les champignons phytopathogènes.

13. Procédé pour la préparation d'une composition agrochimique selon la revendication 6, caractérisé en ce que l'on mélange intimement au moins un composé de formule I selon la revendication 1 avec des additifs solides ou liquides appropriés et/ou des agents de surface.

## Revendications (Revendications pour l'(les) Etat(s) contractant(s) suivant(s): ES)

1. Procédé pour la préparation des composés de formule I 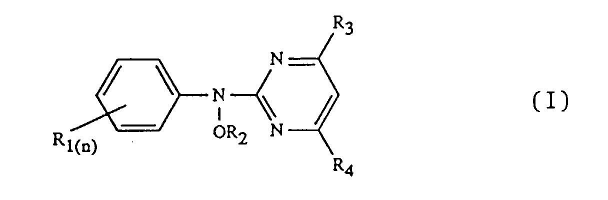 dans laquelle
R₁ représente l'hydrogène, un halogène, le méthyle, le méthoxy ou le trifluorométhyle;
n est égal à 1 ou 2;
R₂ représente l'hydrogène, un alkyle en C₁-C₆, un alkyle en C₁-C₃ substitué par un halogène, le cyano ou le méthoxy ou un alcényle en C₂-C₅, un alcynyle en C₂-C₅, le benzyle, COR₅, CON(R₅)R₆, CSN(R₅)R₆, CO(OR₆), CO(SR₆), CS(SR₅), SO₂R₇, PO(OR₆)₂ ou Si(R₆)₃;
R₃ et R₄ représentent, indépendamment l'un de l'autre, l'hydrogène, le cyclopropyle ou le méthylcyclopropyle et l'un des radicaux R₃ et R₄ peut être également un alkyle en C₁-C₅;
R₅ représente l'hydrogène, un alkyle en C₁-C₆, un alkyle en C₁-C₃ substitué par 1 à 3 halogènes, un alkyle en C₁-C₃ substitué par OR₆ ou SR₆, un alcényle en C₂-C₅, un alcényle en C₂-C₅ substitué par 1 à 3 halogènes, un alcynyle en C₂-C₅, un cycloalkyle en C₃-C₆, le phényle ou un phényle mono-, di- ou trisubstitué par un halogène, un alkyle en C₁-C₃, un halogénoalkyle en C₁-C₃, un alcoxy en C₁-C₃, le nitro ou le cyano;
R₆ représente un alkyle en C₁-C₆;
R₇ représente un alkyle en C₁-C₃, le phényle ou un phényle mono- ou disubstitué par un halogène, un alkyle en C₁-C₂, un halogénoalkyle en C₁-C₂, le méthoxy, le nitro; incluant les sels d'addition avec des acides des composés de formule I, caractérisé en ce que l'on fait réagir :
A) un halogénure de pyrimidinyle de formule II 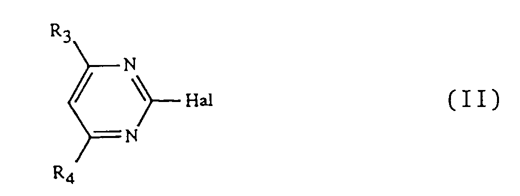 avec une phénylhydroxylamine de formule III 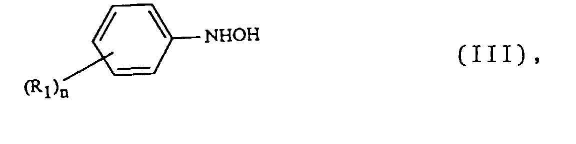 où R₁, R₃, R₄ et n ont les significations indiquées pour la formule I et Hal représente un halogène, de préférence le chlore ou le brome, en présence d'un acide dans des solvants organiques inertes à des températures allant de -30°C à 100°C, de préférence allant de 0°C à 50°C et
B) le composé Ia résultant 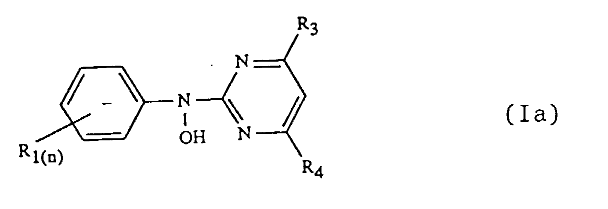
C) avec un composé de formule IV
R₂-X (IV);
où R₁, R₂, R₃, R₄ et n ont les significations indiquées pour la formule I et X représente un halogène, de préférence le chlore ou le brome ou le reste imidazolyle; ou
D) avec un anhydride de formule V
(R₅CO)₂O (V);
ou
E) avec un isocyanate de formule VI
R₄-N=C=Y (VI),
où Y représente l'oxygène ou le soufre, dans des solvants organiques inertes à des températures allant de -20°C à 150°C et, en ce qui concerne les réactions (C) et (D), en présence d'un accepteur d'acide.

2. Procédé selon la revendication 1, caractérisé en ce que l'on prépare les composés de formule I dans laquelle
R₁ représente l'hydrogène, le fluor, le chlore ou le brome;
n est égal à 1;
R₂ représente l'hydrogène, un alkyle en C₁-C₄, un alcényle en C₂-C₃, un alcynyle en C₂-C₃, COR₅, CON(R₅)R₆, CSNR₅(R₆) ou COOR₆;
R₃ et R₄ représentent, indépendamment l'un de l'autre, l'hydrogène, le cyclopropyle ou le méthylcyclopropyle et l'un des radicaux R₃ et R₄ peut être également un alkyle en C₁-C₅;
R₅ représente l'hydrogène, un alkyle en C₁-C₆, un cycloalkyle en C₃-C₆, un alcényle en C₂-C₅, un alcynyle en C₂-C₅, le phényle ou un phényle monosubstitué par le fluor, le chlore, le brome, le méthyle, le trifluorométhyle, le trichlorométhyle, le tribromométhyle, le méthoxy, le nitro ou le cyano;
R₆ représente un alkyle en C₁-C₃.

3. Procédé selon la revendication 1, caractérisé en ce que l'on prépare les composés de formule I dans laquelle
R₁ représente l'hydrogène, le fluor ou le chlore;
n est égal à 1;
R₂ représente l'hydrogène, un alkyle en C₁-C₃, COR₅, CON(R₅)R₆, CSNR₅(R₆) ou COOR₆;
R₃ et R₄ représentent, indépendamment l'un de l'autre, le cyclopropyle ou le méthylcyclopropyle et l'un des radicaux R₃ et R₄ peut être également un alkyle en C₁-C₅;
R₆ représente un alkyle en C₁-C₃.

4. Procédé selon la revendication 1, caractérisé en ce que l'on prépare les composés de formule I suivants:
la N-(4-méthyl-6-cyclopropylpyrimidinyl-2)-phénylhydroxylamine;
la N-(4-méthyl-6-cyclopropylpyrimidinyl-2)-3-fluorophénylhydroxylamine;
la O-acétyl-N-(4-méthyl-6-cyclopropylpyrimidinyl-2)-phénylhydroxylamine;
la O-pivaloyl-N-(4-méthyl-6-cyclopropylpyrimidinyl-2)-phénylhydroxylamine.

5. Composition pour lutter contre ou prévenir une attaque par les microorganismes nuisibles, caractérisée en ce qu'elle contient, comme composant actif, au moins un composé de formule I selon la revendication 1, associé à un support approprié.

6. Composition selon la revendication 5, caractérisée en ce qu'elle contient, comme composant actif, au moins un composé de formule I selon la revendication 2.

7. Composition selon la revendication 5, caractérisée en ce qu'elle contient, comme composant actif, au moins un composé de formule I selon la revendication 3.

8. Composition selon la revendication 5, caractérisée ence qu'elle contient, comme composant actif, au moins un composé de formule I selon la revendication 4.
